# EUROPEAN PATENT APPLICATION

(11) **EP 2 913 017 A1**
(43) Date of publication of application: **02.09.2015**
(21) Application number: 14000698.2
(22) Date of filing: 27.02.2014
(51) Int. Cl.: A61B 18/14, A61B 18/00

(54) **Irrigated ablation catheter**

(71) Applicant: OSYPKA AG., 79618 Rheinfelden-Baden (DE)
(72) Inventor: Röhlig, Claus-Christian, Dr., 79618 Rheinfelden (DE); Göttsche, Thorsten, 79618 Rheinfelden (DE)

(57) **Abstract**

The present invention relates to catheters in general and more particularly to improvements in so-called "irrigated ablation catheter" that may be used in mapping and ablation procedures of tissue, preferably heart tissue in the treatment of cardiac arrhythmia.

The present invention is directed irrigated ablation catheter comprising an elongated catheter shaft (4), a deflectable section (3) distal to the catheter shaft, an ablation catheter tip (1) distal to the deflectable section. The ablation catheter tip comprises an irrigation fluid flow distributor (10) having a distal end (16) and a proximal end (15) whereby the irrigation flow distributor has an inner channel (11) and an outer channel (12) whereby the inner channel leads distally to the outer channel. The outer channel has a helical shape and is in direct contact with a plurality of irrigation fluid exit ports, said exit ports being arranged such that they follow the spiral course of the outer channel.

## Description

The present invention relates to catheters in general and more particularly to improvements in so-called "irrigated ablation catheter" that may be used in mapping and ablation processs of tissue, preferably heart tissue in the treatment of cardiac arrhythmia.

Irrigated catheters are now commonly used in ablation process. Irrigation provides many benefits including cooling of the electrode and tissue which prevents overheating of tissue that can otherwise cause the formation of coagulum.

An ablation catheter typically carries one or more electrodes, which are used for ablation. A common method used for ablation is the radiofrequency (RF) which is accomplished by transmission of radiofrequency energy to a desired target area through an electrode assembly to ablate tissue at the target site. The RF energy delivered through the electrode causes the tissue in contact with the electrode to heat. A problem which may occur is tissue overheating which causes unwanted tissue damage, uncontrolled lesion size and depth and the formation of a coagulum around the RF electrode which in turn reduces the efficiency of the ablation.

Known irrigated ablation catheters are, for example, open ablation catheters which deliver the cooling fluid through holes arranged on the surface of the ablation catheter tip to prevent overheating of the tissue and to achieve deeper lesion and reduced coagulum formation.

US Patent 6611699 describes various catheter designs with irrigated tip electrodes.

When placing the so called "open ablation catheters" to obliterate the area causing the arrhythmia it may happen that the irrigation fluid exit ports arranged on the su rface of the ablation catheter tip are covered or blocked by tissue resulting in a hindered flow of irrigation fluid. The distribution of the cooling fluid in the inner cavity of the ablation electrode influences the temperature distribution on the surface of the electrode.

There is therefore a need to provide an irrigated ablation catheter having an improved cooling system for reducing the overall electrode temperature even if irrigation exit ports on the ablation catheter tip are blocked. It should in particular be ensured that the heat removal from the catheter tip is permanent and continuous during the ablation process so that a homogeneous and effective cooling of the tip surface can be reached and maintained.

Especially desirable is an improved irrigation fluid flow distribution resulting in a homogeneous cooling of the ablation catheter tip thus avoiding the formation of hot spots at the surface of the ablation catheter tip, said tip being in direct contact with the tissue. The irrigation and cooling leads to a deeper and better penetration of the radiofrequency energy, the thermal effects of ablation are thus deeper and the result of ablation procedure is improved.

The present invention is directed irrigated ablation catheter comprising an elongated catheter shaft (4), a deflectable section (3) distal to the catheter shaft, an ablation catheter tip (1) distal to the deflectable section. The ablation catheter tip (1) comprises an irrigation fluid flow distributor (10) having a distal end (16) and a proximal end (15) whereby the irrigation flow distributor has an inner channel (11) and an outer channel (12) whereby the inner channel leads distally to the outer channel. The outer channel (12) has a helical shape and is in direct contact with a plurality of irrigation fluid exit ports (21), said exit ports being arranged such that they follow the spiral course of the outer channel.

In use irrigation (cooling) fluid is pumped through the inner channel (11) leaving the inner channel at the distal end (16) of the irrigation fluid flow distributor (10). The cooling fluid then follows the spiral course of the helical outer channel (12). As the outer channel is in direct contact with a plurality of irrigation fluid exit ports (21, 23) whereby the exit ports (21) are arranged such that they follow the spiral course of the outer channel, the irrigation (cooling) fluid can exit the ablation catheter tip through the exit ports (21, 23) to cool the tissue adjacent to the ablation catheter tip.

The inventive concept is the combination of two structural parts, namely the helical construction of the irrigation outer channel combined with the corresponding position of the irrigation exit ports. Due to this concept, the homogeneity and effectiveness of the tissue cooling is guaranteed. Furthermore this concept allows setting the desired cooling properties. A permanent and continuous heat removal avoiding the formation of hot spots on the surface of the ablation electrode is ensured.

The helical outer channel is a single helix or a double helix.

In preferred embodiment the helical outer channel consists of two helices which are wound around each other thus providing a double helix. Concerning the double helix the water flow of each helix reaches the same cooling point. This may be advantageous.

In one embodiment the cross section of the helical outer channel decreases from its distal to its proximal end to guarantee an optimal and constant flow rate of the irrigation (cooling) fluid as the cooling fluid moves downwards.

Typically the flow rate of the cooling fluid is in the range between 2m/minute and 20ml/min.

Instead of decreasing the cross section, it would also be possible to vary the pitch of the helix.

The term "the outer channel is in direct contact with the irrigation fluid exit ports" encompasses two different embodiments of the ablation catheter tip.

In one embodiment the ablation catheter tip is formed in one piece. The irrigation fluid exit ports are part of the outer channel, are integrated into the outer channel of the irrigation fluid flow distributor.

In a further embodiment the ablation catheter tip is formed in two pieces, said pieces being a cap and the irrigation flow distributor. The cap and the irrigation fluid flow distributor is crafted out of a single piece of material or the cap is tightly slid over the irrigation fluid flow distributor. The cap is adjacent to and in direct contact with the outer channel of the irrigation fluid flow distributor.

The cap and the irrigation fluid flow distributor are in one embodiment made of the same conductive material, for example Au, Pt/Ir, Pt, stainless steel, conductive plastic and the like.

The cap and the irrigation fluid flow distributor are in one embodiment made of different conductive material, for example the cap is made of Au or Pt/Ir and the flow distributor is made of stainless steel.

In a further embodiment only the cap is made of a conductive material such as e.g. Au or Pt/Ir and the fluid flow distributor is made of a nonconductive material such as biologically compatible plastic.

Important is the position and distribution of the irrigation fluid exit ports arranged on the cap. Concerning the cap two areas are differentiated, the side part of the cap and the tip of the cap. In both areas there are irrigation fluid exit ports.

The term "plurality of irrigation fluid exit ports" refers to more than one exit, preferably more than 10 exits, more preferably more than 20 exits, for example 20-40 exits.

The irrigation fluid warms up to during the passage through the catheter device to finally reach body temperature.

The irrigation flow passage is such that cold fluid, which is supplied via the inner channel, passes at first the distal extremity of the flow distributor. Subsequently the cold fluid enters the cavity of the upper part of the cap and exits through the upper exit ports which are in direct and intense contact with the tissue to be ablated thus providing the tissue area of the most intense contact with the fluid which has only minimal warmed up. The cooling fluid then follows the spiral course of the outer channel thus evenly supplying the outer channel with cooling fluid. On each turn of the spiral course there are exit ports for the exit of the cooling fluid.

In case of exit ports being blocked by tissue, the heat removal is ensured by the cooling fluid following the spiral course of the outer channel, thus forcing the steady cooling of the inner surface of the electrode cap.

These and other features and advantages of the present invention will be better understood by reference to the following description when considered in conjunction with the accompanying drawings wherein:
FIG. 1 shows an ablation catheter device having the inventive irrigated ablation electrode.
Fig. 2A is a side cross sectional view of the catheter shaft having the inventive irrigated ablation electrode.
Fig. 2B is a cross sectional view of the catheter shaft taken along the line A-A of Fig. 2A.
Fig. 3A and Fig. 3B is a side view of the helical irrigation fluid flow distributor.
Fig. 4A and Fig. 4B is a side view of the cap.
Fig. 5A and Fig. 5B show the ablation catheter tip mounted on an adapter.

The numbering of the figures is as follows
- 1: Ablation catheter tip
- 2: Sensing electrode
- 3: Deflectable section
- 4: Catheter shaft
- 5: Fluid source
- 6: Energy source
- 7: Catheter handle
- 8: Electric conductive wire
- 9: Taut wire
- 10: Irrigation fluid flow distributor
- 11: Inner channel
- 12: Outer channel
- 14: Adapter
- 15: Proximal end of the irrigation fluid flow distributor
- 16: Distal end of the irrigation fluid flow distributor
- 20: cap
- 21: Exit ports in the side area
- 22: Temperature sensor
- 23: Exit ports in the tip area

### Description of the figures

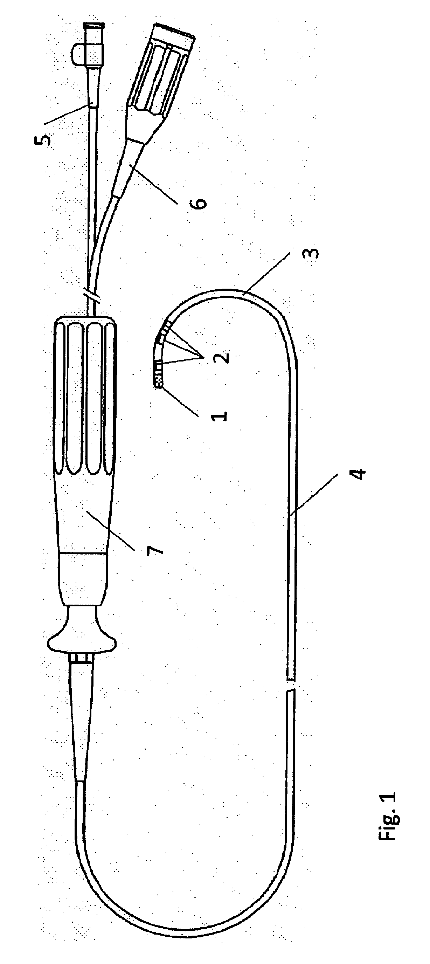

FIG. 1 shows an ablation catheter device having the inventive irrigated ablation electrode. The catheter comprises an elongated catheter shaft (4) a deflectable section (3) distal to the catheter shaft (4), an irrigated ablation electrode (1) which is positioned distal to the deflectable section (3). In use the ablation electrode is in direct and intense contact with the tissue to be ablated. Optionally there are one or more sensing electrodes (2) positioned on the deflectable section (3). Catheter handle (7) supports the proximal end of the catheter shaft (4). The energy source (6) is connected to the catheter handle (7) to supply energy to the ablation electrode (1). A fluid source (5) is attached to the catheter handle (7) to apply cooling fluid. The ablation catheter device is a device commonly used in ablation therapy. New and inventive is the design of the ablation electrode (1).

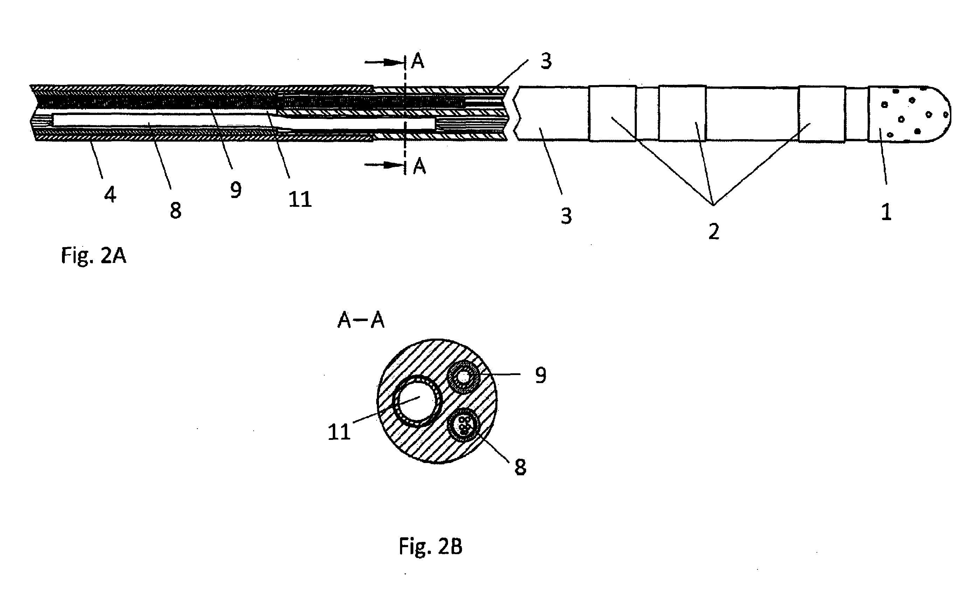

**Fig. 2A** shows the catheter shaft (4) and the deflectable section (3) having the inventive irrigated ablation electrode (1) and in addition sensing electrodes (2). The side cross-sectional view of the catheter shaft shows the electric conduction wires (8), an inner channel (10) for delivering the cooling fluid (irrigation fluid), a taut wire (9) for converting the ablation catheter from a retracted operation mode into an expanded operation mode.
**Fig. 2B** is a cross sectional view of the catheter shaft taken along the line A-A of Fig. 2A showing the tube (10) for delivering the cooling fluid, the taut wires (9), the electrical conduction wires (8).

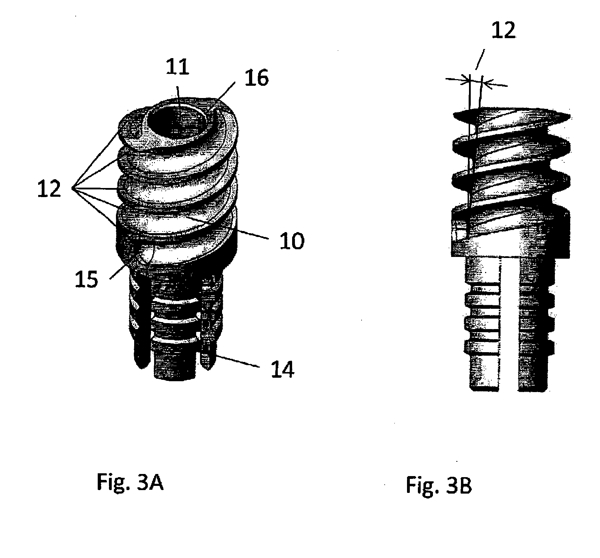

**Fig. 3A** and **Fig. 3B** is a side view of the helical irrigation fluid flow distributor (10)
**Fig. 3A** is an isometric view of the irrigation fluid flow distributor (10). The inner channel (11) delivering the irrigation fluid leads distally to the helical outer channel (12) which is in shape of a double helix. In use the irrigation fluid runs through the inner channel, leaves the inner channel at its distal end (16) and follows the spiral course of the outer channel starting at its distal end and running to its proximal end (15). The flow distributor (10) is mounted via an adapter (14) to the deflectable section (3) of the catheter shaft (4).
**Fig. 3B** is a side view of the irrigation flow distributor (10). Fig. 3B shows in addition to Fig. 3A that the cross section of the helical outer channel decreases from its distal to its proximal end.

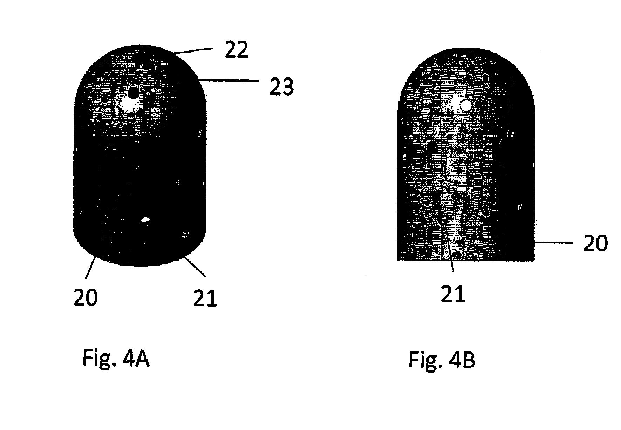

**Fig. 4A** and **Fig. 4B** is a side view of the cap (20). A plurality of irrigation fluid exit ports (21) are arranged on the cap wherein the exit ports are arranged such that they follow the spiral course of the outer channel of the irrigation flow distributor which is positioned inside the cap (not shown in this figure.) In the isometric view **4A** a temperature sensor (22) is positioned within the distal portion of the tip of the cap.

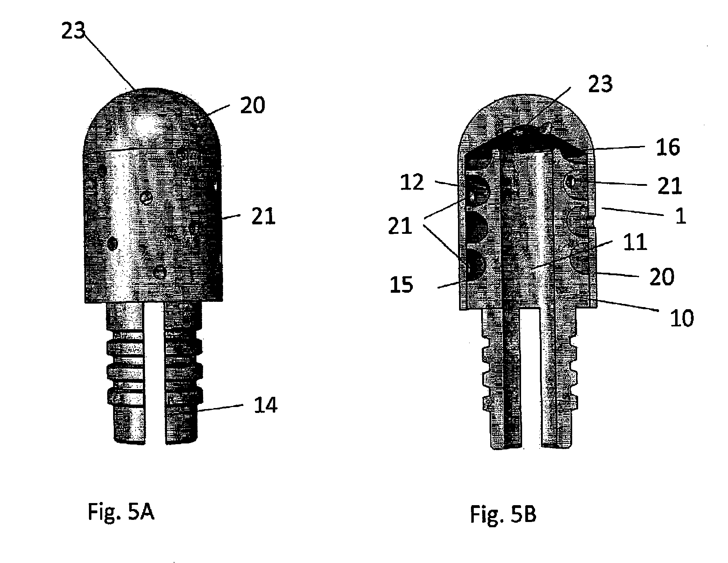

**Fig. 5A** and **Fig. 5B** show the ablation catheter tip (1) mounted on adapter (14).
**Fig. 5A** is a side view showing the ablation catheter tip (1) mounted on adapter (14). The ablation catheter tip is as shown in Fig. 4b
**Fig. 5B** is a cross sectional view of the inventive ablation catheter tip (1) formed in two pieces, a cap (20) and an irrigation fluid flow distributor (10) having a distal end (16) and a proximal end (15). The flow distributor has an inner channel (11) and an outer channel (12). The inner channel (11) leads distally to the outer channel (12). The outer channel is in shape of a double helix having two turns. Cap (20) is tightly slid over the irrigation flow distributor. The irrigation fluid exit ports are arranged on the cap. There are exit ports (23) arranged on the tip of the cap and exit ports (21) arranged such that they follow the spiral course of the outer channel. The cross section of the helical outer channel decreases towards the proximal end.

## Claims

1. Irrigated ablation catheter comprising:
an elongated catheter shaft (4),
a deflectable section (3) distal to the catheter shaft,
an ablation catheter tip (1) distal to the deflectable section,
the ablation catheter tip (1) comprises an irrigation fluid flow distributor (10) having a distal end (16) and a proximal end (15) whereby the irrigation flow distributor (10) has an inner channel (11) and an outer channel (12), whereby the inner channel leads distally to the outer channel, **characterized in that** the outer channel (12) has a helical shape and is in direct contact with a plurality of irrigation fluid exit ports (21), said exit ports being arranged such that they follow the spiral course of the outer channel.

2. Irrigated ablation catheter according to claim 1, whereby the cross section of the helical outer channel decreases from its distal to its proximal end.

3. Irrigated ablation catheter according to claim 1-2, whereby the helical shape is a single helix or a double helix.

4. Irrigated ablation catheter according to claim 3, whereby the helical shape is a double helix.

5. Irrigated ablation catheter according to any one of claims 1-4, whereby the ablation catheter tip (1) is formed in one piece.

6. Irrigated ablation catheter according to any one of claims 1-4, whereby the ablation catheter tip (1) is formed in at least two pieces said pieces being a cap (20) and the irrigation flow distributor (10).

7. Irrigated ablation catheter according to claim 6, whereby the irrigation fluid exit ports (21) are arranged on a cap (20) said exit ports being arranged such that they follow the spiral course of the outer channel.

8. Irrigation ablation catheter according to claim 7, whereby the cap (20) has further irrigation fluid exit ports (23) which are arranged on the tip of the cap.

9. Irrigated ablation catheter according to any one of claims 6-8, whereby the cap (20) is tightly slid over an irrigation fluid flow distributor (10).

10. Irrigated ablation catheter according to any one of claims 6-9, whereby the cap (20) and the irrigation fluid flow distributor (10) are made of the same conductive material.

11. Irrigated ablation catheter according to anyone of claims 6-9, wherein the cap (20) and the irrigation fluid flow distributor (10) are made of different conductive material.

12. Irrigated ablation catheter according to claim 11, whereby the cap (20) is made of Au or Pt/Ir and the irrigation fluid flow distributor (10) is made of stainless steel.

13. Irrigated ablation catheter according to anyone of claims 6-9, wherein the cap (20) is made of a conductive material and the irrigation fluid flow distributor (10) is made of a non-conductive material.

## Amended claims

### Amended claims in accordance with Rule 137(2) EPC.

1. Irrigated ablation catheter comprising:
an elongated catheter shaft (4),
a deflectable section (3) distal to the catheter shaft,
an ablation catheter tip (1) distal to the deflectable section,
the ablation catheter tip (1) comprises an irrigation fluid flow distributor (10) and a cap,
the flow distributor (10) having a distal end (16) and a proximal end (15) whereby the irrigation flow distributor (10) has an inner channel (11) and an outer channel (12),
whereby the distal end of the Inner channel is coupled to the distal end of the outer channel, **characterized In that** the outer channel (12) has a helical shape in form of a double helix and is defined by the cap (20) and the flow distributor whereby the cap (20) Is tightly slid over the irrigation fluid flow distributor (10), the outer channel (12) is in direct contact with a plurality of irrigation fluid exit ports (21), whereby the irrigation fluid exit ports (21) are arranged on the cap (20), said exit ports being arranged such that they follow the spiral course of the outer channel and whereby the cap (20) has further irrigation fluid exit ports (23) which are arranged on the tip of the cap.

2. Irrigated ablation catheter according to claim 1, whereby the cross section of the helical outer channel decreases from its distal to its proximal end.

3. Irrigated ablation catheter according to claim 1 or 2, whereby the cap (20) and the irrigation fluid flow distributor (10) are made of the same conductive material.

4. Irrigated ablation catheter according to claim 1 or 2, wherein the cap (20) and the irrigation fluid flow distributor (10) are made of different conductive material.

5. Irrigated ablation catheter according to claim 4, whereby the cap (20) Is made of Au or Pt/Ir and the irrigation fluid flow distributor (10) is made of stainless steel.

6. Irrigated ablation catheter according to claim 1-2, wherein the cap (20) Is made of a conductive material and the irrigation fluid flow distributor (10) is made of a non-conductive material.
